(19) Europäisches Patentamt
European Patent Office
Office européen des brevets

(11) Publication number: 0 055 999 B1

(12) # EUROPEAN PATENT SPECIFICATION

(45) Date of publication of patent specification: 03.10.84

(51) Int. Cl.³: C 07 C 172/00, A 61 K 31/59

(21) Application number: 82200223.4

(22) Date of filing: 14.02.80

(60) Publication number of the earlier application in accordance with Art. 76 EPC: 0 015 122

(54) ## Vitamin D3.

(30) Priority: 15.02.79 JP 15420/79
04.05.79 JP 54154/79
14.06.79 JP 73956/79
26.07.79 JP 94183/79
02.08.79 JP 98106/79

(43) Date of publication of application:
14.07.82 Bulletin 82/28

(45) Publication of the grant of the patent:
03.10.84 Bulletin 84/40

(84) Designated Contracting States:
CH DE FR GB IT NL SE

(56) References cited:
GB-A-2 006 216

CHEMICAL ABSTRACTS, volume 86, no. 13, 28th March 1977, page 378, abstract 90145d COLUMBUS OHIO (US)

(73) Proprietor: TEIJIN LIMITED
11 Minami Honmachi 1-chome Higashi-ku
Osaka-shi Osaka-fu (JP)

(72) Inventor: Nishikawa, Osamu
28-1-201, Ozu-machi 1-chome
Iwakuni-shi Yamaguchi-ken (JP)
Inventor: Ishimaru, Kenji
92-21, Minamiiwakuni-cho 2-chome
Iwakuni-shi Yamaguchi-ken (JP)
Inventor: Takeshita, Toru
18-4, Tamadaira 3-chome
Hino-shi Tokyo (JP)
Inventor: Tsuruta, Hideki
28-6-305, Ozu-machi 1-chome
Iwakuni-shi Yamuguchi-ken (JP)

(74) Representative: Votier, Sidney David
CARPMAELS & RANSFORD 43, Bloomsbury Square
London WC1A 2RA (GB)

EP 0 055 999 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to active vitamin $D_3$ materials, and to the preparation thereof. More especially the invention relates to novel 24-oxocholecalciferol derivatives, to their preparation, and to their use in pharmaceutical compositions. This application is divided from European Application 80300426.6 (EP—A—15122).

According to the present invention we provide 25-hydroxy-24-oxocholecalciferol of formula [I]

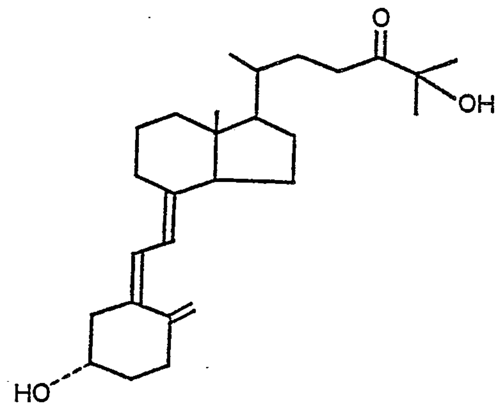

[I]

and 1$\alpha$,25-dihydroxy-24-oxocholecalciferol of formula [2]

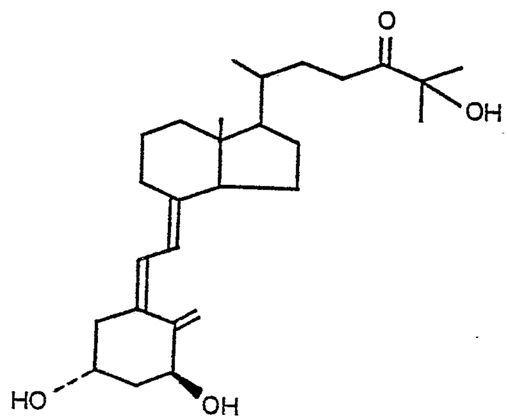

[2]

25-Hydroxy-24-oxocholecalciferol and 1$\alpha$,25-dihydroxy-24-oxocholecalciferol are new compounds that have never been described in any literature and the process for preparation and the biological activities thereof have not been known.

According to the research conducted by the present inventors, it has been found that the novel 25-hydroxy-24-oxocholecalciferol and 1$\alpha$,25-dihydroxy-24-oxocholecalciferol have excellent pharmaceutical effects as agents for controlling the calcium metabolism of warmblooded animals and that the pharmaceutical effects are superior to those of conventional vitamin $D_3$, as illustrated in the detailed animal test described in the examples later.

The novel compounds of this invention may be obtained by treatment of the 25-hydroxy-24-oxocholestane derivatives which are claimed in the parent Application 80300426.6 (EP—A—15122). For example, 25-hydroxy-24-oxocholecalciferol is obtained by irradiation with light and thermally isomerizing 25-hydroxy-24-oxocholesta-5,7-diene expressed by formula [I-ii] given in the parent Application.

1$\alpha$,25-Dihydroxy-24-oxocholecalciferol is prepared likewise from 1$\alpha$,25-dihydroxy-24-oxocholesta-5,7-diene expressed by formula [I-i-I] given in the parent Application.

The ultraviolet rays used in the irradiation of the cholestadiene compounds are usually employed in the wavelength range of about 200 to 360 nm, and preferably from 260 to 310 nm in the present invention. A hydrocarbon or halogenated hydrocarbon, such as hexane, heptane, cyclohexane, benzene, toluene, xylene, carbon tetrachloride, 1,2-dichloroethane, 1,2-dibromomethane, an ether, such as diethyl ether, tetrahydrofuran, dioxane, or an alcohol, such as methanol, ethanol, propanol, hexanol, cyclohexanol, are preferably used as reaction solvent.

The irradiation with ultraviolet rays is conducted at a temperature in the range of —20°C to 80°C, preferably from —10 to 20°C in the absence of oxygen, for example in an argon or nitrogen atmosphere.

Thus, the irradiation with ultraviolet rays causes cleavage between the 9- and 10-positions in the 5,7-diene compound used as starting material to afford 25-hydroxy-25-oxoprevitamin $D_3$ or 1$\alpha$,25-dihydroxy-24-oxoprevitamin $D_3$.

The resulting previtamin $D_3$ is isomerized to 25-hydroxy-24-oxocholecalciferol or 1$\alpha$,25-dihydroxy-24-oxocholecalciferol of aforementioned formulae [I] and [2] respectively.

The temperature during the isomerization is preferably in the range of 10—120°C. Usually, the isomerization is preferably effected in an inert organic solvent, which in practice is the same solvent as that used in the irradiation with ultraviolet rays.

Thus, novel 25-hydroxy-24-oxocholecalciferol or 1$\alpha$,25-dihydroxy-24-oxocholecalciferol are obtained.

Resultant 25-hydroxy-24-oxocholecalciferol and 1$\alpha$,25-dihydroxy-24-oxocholecalciferol have, as shown in the tests described below, a high pharmaceutical activity of promoting intestinal calcium absorption and raising the calcium concentration in blood.

Therefore, the above active vitamin $D_3$ prepared in accordance with the present invention can be used as a drug applicable to diseases caused by abnormal calcium metabolism.

Suitable dosages of the novel 25-hydroxy-24-oxocholecalciferol or 1$\alpha$,25-dihydroxy-24-oxocholecalciferol in clinical application, based on the results of pharmacological tests conducted by the present inventors, have been found to be about 0.04—0.4 $\mu$g (about 96—960 p mole) per kilogram of the body weight of a warm-blooded animal.

The active vitamin $D_3$ of the present invention can be clinically or veterinarily applied to the following diseases:

vitamin D dependent rickets, renal osteodystrophy, hypoparathyroidism, osteoporosis, osteomalacia, Behcet's disease, malabsorption syndrome, hypocalcemia induced by liver cirrhosis, hypocalcemia induced by steatorrhoea, hypocalcemia caused by vitamin D resistant rickets, and abnormal calcium and phosphorus metabolism caused by liver failure, renal failure, gastrointestinal tract failure or parathyroid failure and related bone diseases.

Further, a composition containing 25-hydroxy-24-oxocholecalciferol or 1$\alpha$,25-dihydroxy-24-oxocholecalciferol can be used in combination with other calcium metabolism regulating agents. For example, it can be applied to the treatment of Behcet's disease in combination with calcitonin.

Suitable routes of dosing include oral, buccal, and parenteral, intramuscular, subcutaneous, intravenous, and intrarectal administration. Dosage forms are, for example, compressed tablets, coated tablets, hard or soft elastic gelatin capsules, ethyl alcohol solutions, oil solutions, and aqueous suspensions.

The solvent for the oil solutions may be a vegetable oil such as corn, cotton-seed, coconut, almond or peanut oil, a fish liver oil, or an oily ester such as polysorbate 80.

For intrarectal administration, the above active vitamin $D_3$ may be formed into a pharmaceutical composition containing a suppository base such as cacao butter or other triglycerides. To prolong the shelf life of the pharmaceutical composition, it may advantageously include an antioxidant such as ascorbic acid, butylated hydroxyanisole, or hydroquinone.

The active vitamin $D_3$ according to this invention can be mixed with a feed for domestic animals and the feed composition for domestic animals which contains the compound can be used, in amounts not to cause toxicity, for the prevention of hypocalcemia of cows at or near the time or delivery, or the prevention of hypocalcemia of domestic animals with no history of hypocalcemia. When such compositions are administered to poultry during oviposition, it is posible to prevent them from soft-shell eggs, which constitutes another characteristic feature of the active vitamin $D_3$ of the present invention.

Moreover, the novel 25-hydroxy-24-oxocholecalciferol and 1$\alpha$,25-dihydroxy-24-oxocholecalciferol are useful also as intermediates for the preparation of active vitamin $D_3$ 24,25-dihydroxycholecalciferol and 1$\alpha$,24,25-trihydroxycholecalciferol. Thus, the present inventors have now succeeded in the preparation of 24,25-dihydroxychlolecalciferol and 1$\alpha$,24,25-trihydroxycholecalciferol by reducing the oxo group at the 24-position in the above 25-hydroxy-24-oxocholecalciferol and 1$\alpha$,25-dihydroxy-24-oxocholecalciferol respectively.

The reduction adopted to reduce the oxo group (=O) at the 24-position of the above compounds can be conducted under any conditions which selectively reduce the oxo group (=O) at the 24-position without bringing about the reduction of double bonds.

As for such reduction conditions for instance, there are the Ponndorf reduction in which aluminium-alkoxide is used, the Birch reduction which is conducted in liquid ammonia or amine in which lithium or sodium is used, and other reduction methods in which a reduction reagent is used to give a hydrogen anion, such as aluminium hydrides, boron hydrides. Of these, the methods wherein a reduction reagent such as an aluminium or boron hydride is used, are preferable because of their simplicity of operation.

As for the aluminium hydrides, for instance, lithium aluminium hydride and sodium aluminium hydride may be mentioned, and as for the boron hydrides, for instance, sodium boron hydride and lithium boron hydride may be mentioned.

It is preferable to use 1.5—4 moles of such reduction reagent as aluminium hydride, boron hydride for each mole of the aforementioned 24-oxocholecalciferol derivatives.

As to solvents for use in the reduction, there may be mentioned ether solvents such as diethyl ether and tetrahydrofuran, alcohols such as methyl alcohol, ethyl alcohol and isopropyl alcohol, and other solvents such as dimethyl formamide and dimethyl sulfoxide.

The reaction temperature is preferably in the range of 5 to 50°C and the reaction is usually completed in several hours.

The following Examples are given for the purpose of illustrating the invention.

Example 1

*Synthesis of 25-hydroxy-24-oxocholecalciferol*

157 mg of 3$\beta$,25-dihydroxy-24-oxocholesta-5,7-dien, produced in accordance with Example 8 (iv) of the parent Application was dissolved in a mixture of ethyl alcohol (50 ml) and ether (500 ml), and this solution was irradiated through a vycol filter with ultraviolet rays for 14 minutes at 5°C in an atmosphere or argon using a 200 W high pressure mercury lamp (654A—36, Trade Mark for a product of Hanovia Company).

After the reaction, ether was evaporated off at about 30°C under reduced pressure, 250 ml of benzene was added to the concentrated solution, and isomerization was carried out for 2.5 hours under reflux of benzene in an atmosphere of argon.

The reaction mixture was concentrated. The resulting residue was carefully separated by preparative thin-layer chromatography using a silica gel carrier which was immersed in a solution of silver nitrate in acetonitrile to impregnate it in an amount of about 2% weight as silver nitrate and an eluting solvent consisting of methyl alcohol and chloroform.

There were obtained 8.7 mg of 25-hydroxy-24-oxo previtamin $D_3$ and 14.8 mg of 25-hydroxy-24-oxocholecalciferol having the following characteristics.

*25-hydroxy-24-oxo previtamin $D_3$

UV (EtOH); $\lambda_{max}$ 260

*25-hydroxy-24-oxocholecalciferol

UV (EtOH)nm; $\lambda_{max}$ 264.5 ($\varepsilon$=15500), $\lambda_{min}$ 228 ($\varepsilon$=8400)

IR (neet) cm$^{-1}$; 3375, 2925, 2860, 1705, 1385, 1045

NMR ($CDCl_3$,TMS), $\delta$ (ppm); 0.54 (3H, S, C—18—$CH_3$), 1.37 (6H, S, C—25—$CH_3$), C—27—$CH_3$), 3.90 (1H, b, C—3—H), 4.30, 5.03 (2H, m, C—19—H x 2), 6.01, 6.20 (2H, AB quartet, J=11.5, C—6—H, (C—7—H).

High resolution mass spectrum

$M^+$; 414.3131 ($C_{27}H_{42}O_3$)

Example 2

*Synthesis of 24,25-dihydroxycholecalciferol*

4.9 mg of 25-hydroxy-24-oxocholecalciferol was dissolved in 1 ml of methyl alcohol.

To the solution, 0.76 mg of sodium boron hydride was added. The mixture was stirred at room temperature for 20 minutes. After the reaction water was added, and the aqueous solution was extracted with ether. The ether extract was washed with a saturated aqueous solution of sodium chloride, dried over anhydrous sodium sulfate, filtered and concentrated. There was obtained about 5 mg of 24,25-dihydroxycholecalciferol (quantitative yield).

This product showed the same analytical-thin-layer chromatography and high pressure liquid chromatography data as the authentic sample, and was identified as 24,25-dihydroxycholecalciferol from the following spectrum data.

UV (Ether)nm; $\lambda_{max}$ 264, $\lambda_{min}$ 228

High resolution mass spectrum

$M^+$: 416.3413 ($C_{27}H_{44}O_3$)

Example 3

*Synthesis of 1$\alpha$,25-dihydroxy-24-oxocholecalciferol.*

70 mg of 1$\alpha$,3$\beta$,25-trihydroxy-24-oxocholesta-5,7-dien, prepared in accordance with Example 9(iv) of the parent Application, was dissolved in a mixture of ethyl alcohol (50 ml) and ether (500 ml), and this solution was irradiated through a vycol filter with ultraviolet rays for 7 minutes at about 10°C in an atmosphere of argon using a 200 W high pressure mercury lamp (654A—36, Trade Mark for a product of Hanovia Company).

After the reaction, ether was evaporated off at about 30°C under reduced pressure. 250 ml of benzene was added to the concentrated solution, and isomerization was carried out for 2.5 hours under reflux of benzene in an atmosphere of argon.

The reaction mixture was concentrated. The resulting residue was carefully separated successively by preparative thin-layer chromatography using a silica gel carrier which was immersed in a solution of silver nitrate in acetonitrile to impregnate it in an amount of about 2% by weight of silver nitrate, and an eluting solvent consisting of a mixture of methyl alcohol and dichloromethane, and by preparative thin-layer chromatography using a silica gel carrier and an eluting solvent consisting of a mixture of benzene and acetone.

There was obtained 10.8 mg of 1$\alpha$,25-dihydroxy-24-oxocholecalciferol having the following characteristics.

Melting point; 91—93.5°C

IR (KBr) $cm^{-1}$; 3450, 2960, 1715, 1390, 1055

NMR ($CDCl_3$, TMS), $\delta$ (ppm); 0.55 (3H, S, C—18—$CH_3$), 1.37 (6H, S, C—26—$CH_3$, C—27—$CH_3$), 4.98, 5.30 (2H, m, C—19—H x 2), 6.18 (2H, AB quartet, J=11.5 Hz, C—6—H, C—7—H)

UV (Ether)nm; $\lambda_{max}$ 264.5, $\lambda_{min}$ 228.5

High resolution mass spectrum;

$M^+$: 430.3116 ($C_{27}H_{42}O_4$)

Example 4

*Effect of 25-hydroxy-24-oxocholecalciferol to promote calcium absorption from the intestinal tract.*

Comparison with 1$\alpha$,25-dihydroxy cholecalciferol;—

Weanling Wistar male rats (with a body weight of about 100g) which had been fed only with vitamin D-deficient diet for 6 weeks were fasted overnight. A solution of 25-hydroxy-24-oxocholecalciferol (250 ng/head) in a 1:1 mixture of ethyl alcohol and physiological saline solution or a solution of 1$\alpha$,25-dihydroxy cholecalciferol (250 ng/head) in the same mixture was intraveneously administered to the rats. They were killed 4 hours, 8 hours, 24 hours and 48 hours later and calcium absorption at the intestinal tube was measured by the everted gut sac method [see Martin, D. L. and Deluca, H. F., Amer, J. Physiol, *216*, 1351 (1969)].

The results are shown in Fig. 1.

It is seen from the experimental results that 1$\alpha$,25-dihydroxy cholecalciferol showed that maximum effect after about 8 hours from administration and the effect decreased markedly from then, while 25-hydroxy-24-oxo-cholecalciferol showed the effect after about 4 hours from administration and the effect continued for 44 hours.

Example 5

Combination of 1$\alpha$,25-dihydroxy-24-oxocholecalciferol with the 1$\alpha$,25-dihydroxy cholecalciferol-receptor in the chick's intestinal tube:—

It is well known that the combination ability of vitamin D analogue with the 1$\alpha$,25-dihydroxy cholecalciferol-receptor is relative to the strength of the effect to promote calcium absorption from the intestinal tract. And so the combination ability of 1$\alpha$,25-dihydroxy-24-oxo cholecalciferol with the 1$\alpha$,25-dihydroxy cholecalciferol-receptor in the chick's intestinal tube was investigated by the authentic method [see, for example, Steroids. 30, 2, 245—257 (1977)].

There were obtained the results shown in Table 1.

TABLE 1

| Vitamin $D_3$ analogue | 50% Displacement (pg) | Molar ratio |
|---|---|---|
| 1$\alpha$,25-dihydroxy cholecalciferol | 46 | 1 |
| 1$\alpha$,25-dihydroxy 24-oxo-cholecalciferol | 138 | 3 |

It is seen from these results that 1$\alpha$,25-dihydroxy-24-oxocholecalciferol was expected to have the active vitamin $D_3$-effects.

## Claims

1. 25-hydroxy-24-oxocholecalciferol of formula [1]

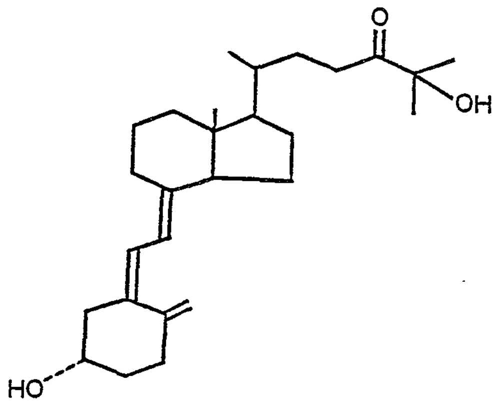

[1]

and 1$\alpha$,25-dihydroxy-24-oxocholecalciferol of formula [2]

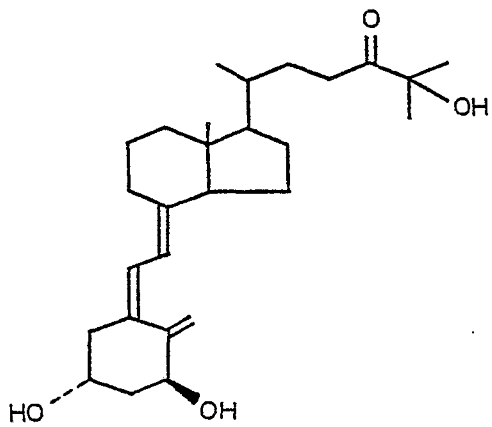

[2]

2. The compounds of claim 1, for use as pharmaceutically active agents.
3. A process for the preparation of 25-hydroxy-24-oxocholecalciferol which comprises irradiating with light 25-hydroxy-24-oxocholesta-5,7-diene and thermally isomerising the resulting previtamin $D_3$.
4. A process for the preparation of 1$\alpha$,25-dihydroxy-24-oxocholecalciferol which comprises irradiating with light 1$\alpha$,25-dihydroxy-24-oxocholesta,57-diene and thermally isomerising the resulting previtamin $D_3$.
5. A process as claimed in claim 3 or 4, in which the irradiation is effected with ultraviolet rays of wavelength in the range 200 to 360 nm.
6. A process as claimed in any of claims 3 to 5 in which the irradiation is conducted at a temperature in the range −20 to 80°C.
7. A process as claimed in any of claims 3 to 6 in which the isomerisation is effected at a temperature in the range 10—120°C.
8. A process for producing 25,24-dihydroxy-cholecalciferol which comprises reducing the oxo group at the 24-position of the compound of formula [1] given in claim 1.
9. A process for producing 1$\alpha$,24,25-trihydroxycholecalciferol which comprises reducing the oxo group at the 24-position of the compound of formula [2] given in claim 1.
10. A pharmaceutically active composition comprising a compound as claimed in claim 1.

**Patentansprüche**

1. 25-Hydroxy-24-oxocholecalciferol der Formel (1)

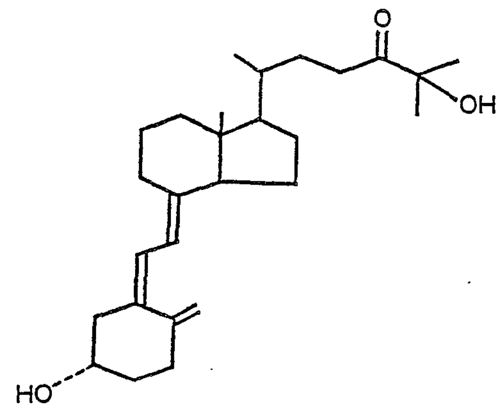

[1]

und 1$\alpha$,25-dihydroxy-24-oxocholecalciferol der Formel (2)

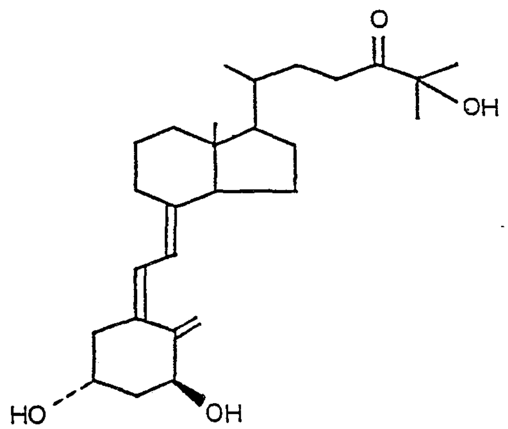

[2]

2. Verbindungen von Anspruch 1 zur Verwendung als pharmazeutisch aktive Mittel.

3. Verfahren zur Herstellung von 25-Hydroxy-24-oxocholecalciferol durch Bestrahlen mit Licht von 25-Hydroxy-24-oxocholesta-5,7-dien und thermisches Isomerisieren des entstehenden Prävitamins $D_3$.

4. Verfahren zur Herstellung von 1$\alpha$,25-Dihydroxy-24-oxocholecalciferol durch Bestrahlen mit Licht von 1$\alpha$,25-Dihydroxy-24-oxocholestra-5,7-dien und thermisches Isomerisieren des entstehenden Prävitamins $D_3$.

5. Verfahren nach Anspruch 3 oder 4, dadurch gekennzeichnet, daß die Bestrahlung durchgeführt wird mit Ultraviolettstrahlen einer Wellenlänge im Bereich von 200 bis 360 nm.

6. Verfahren nach einem der Ansprüche 3 bis 5, dadurch gekennzeichnet, daß die Bestrahlung bei einer Temperatur im Bereich von —20 bis 80°C ausgeführt wird.

7. Verfahren nach einem der Ansprüche 3 bis 6, dadurch gekennzeichnet, daß die Isomerisierung durchgeführt wird bei einer Temperatur im Bereich von 10 bis 120°C.

8. Verfahren zur Herstellung von 24,25-Dihydroxy-cholecalciferol durch Reduzieren der Oxogruppe in der 24-Position der Verbindung von Formel (1) von Anspruch 1.

9. Verfahren zur Herstellung von 1$\alpha$,24,25-Trihydroxycholecalciferol durch Reduzieren der Oxogruppe in der 24-Position der Verbindung von Formel (2) von Anspruch 1.

10. Pharmazeutische aktive Zusammensetzung, enthaltend eine Verbindung aus Anspruch 1.

## Revendications

1. 25-Hydroxy-24-oxocholécalciférol de formule [1]

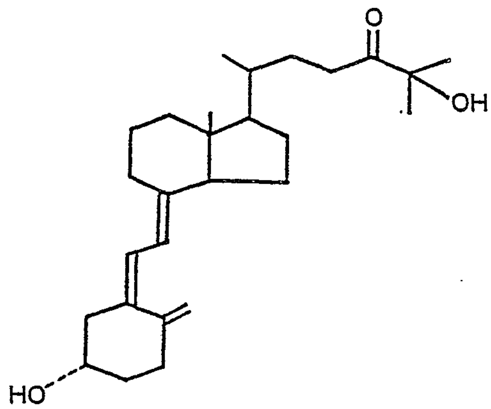

[1]

et 1$\alpha$,25-dihydroxy-24-oxocholécalciférol de formule [2]

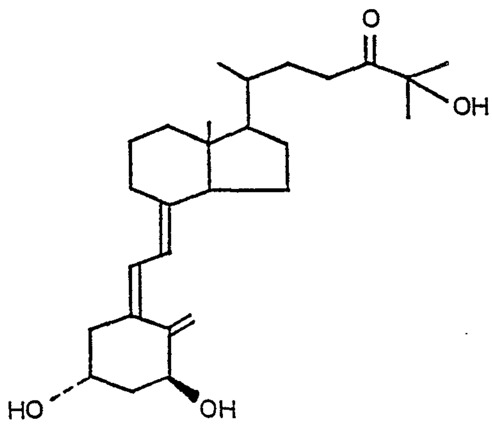

[2]

2. Les composés de la revendication 1 en vue de l'utilisation en tant qu'agents pharmaceutiquement actifs.

3. Un procédé pour la préparation de 25-hydroxy-24-oxocholécalciférol consistant à irradier du 25-hydroxy-24-oxocholesta-5,7-diène avec de la lumière et à isomériser thermiquement la prévitamine $D_3$ résultante.

4. Procédé pour la préparation de 1$\alpha$,25-dihydroxy-24-oxocholécalciférol consistant à irradier du 1$\alpha$,25-dihydroxy-24-oxocholesta-5,7-diène avec de la lumière et à isomériser thermiquement la prévitamine $D_3$ résultante.

5. Procédé selon revendication 3 ou 4, caractérisé en ce que l'irradiation s'effectue à l'aide de rayons ultraviolets de longueur d'onde comprise entre 200 et 360 nm.

6. Procédé selon l'une quelconque des revendications 3 à 5, caractérisé en ce que l'irradiation est conduite à une température dans la gamme de —20 à 80°C.

7. Procédé selon l'une quelconque des revendications de 3 à 6, caractérisé en ce que l'isomérisation s'effectue à une température se situant dans l'intervalle de 10 à 120°C.

8. Procédé pour la production de 24,25-dihydroxy-cholécalciférol qui consiste à réduire le groupe oxo en position 24 du composé de formule [1] présenté dans la revendication 1.

9. Procédé pour la production de 1$\alpha$,24,25-trihydroxy-cholécalciférol qui consiste à réduire le groupe oxo en position 24 du composé de formule [2] présenté dans la revendication 1.

10. Composition pharmaceutiquement active caractérisée en ce qu'elle renferme un composé selon la revendication 1.

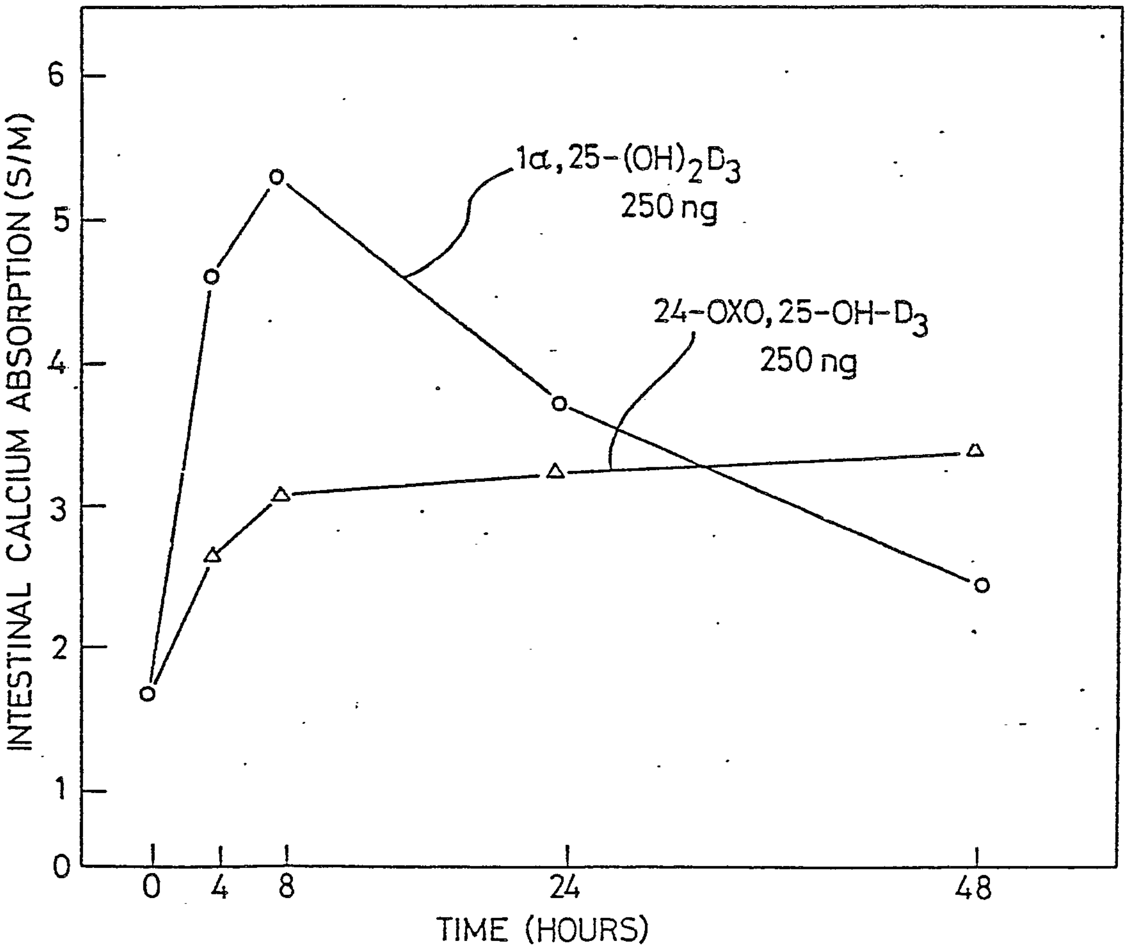
6
5
4
3
2
1
0
INTESTINAL CALCIUM ABSORPTION (S/M)
1α,25-(OH)2D3
250 ng
24-OXO,25-OH-D3
250 ng
0
4
8
24
48
TIME (HOURS)